# EUROPEAN PATENT APPLICATION

(11) **EP 4 567 823 A1**
(43) Date of publication of application: **11.06.2025**
(21) Application number: 23214066.5
(22) Date of filing: 04.12.2023
(51) Int. Cl.: G16H 20/30, A61B 5/00, A61B 5/11, G06F 3/01

(54) **COMPUTER-IMPLEMENTED AUTHORING AND PERFORMING PHYSICAL REHABILITATION EXERCISES IN MIXED REALITY**

(71) Applicant: Vietnam National University Ho Chi Minh City, Ho Chi Minh City (VN)
(72) Inventor: LE, Khanh-Duy, Ho Chi Minh City (VN); TRAN, Minh-Triet, Ho Chi Minh City (VN)
(74) Representative: Cross, James Peter Archibald

(57) **Abstract**

A mixed-reality (MR) system allows a rehabilitator to define a variety of physical exercises by virtually placing virtual objects in a space with suitable attributes to control different parameters of the exercises according to the patient's treatment process. The patient may then perform the authored exercises in MR by interacting with the virtual objects using the suitable body parts following the rehabilitator's instruction. The rehabilitator does not need to accompany the patient during the entire practicing time. The exercise practiced by the patient may be recorded and transferred to the rehabilitator's computing device (e.g. PC or tablet) for remote monitoring. The rehabilitator thus can work with more patients at a time than in the conventional in-person 1-1 setting. The system can also precisely record the patient's exercise performance and the rehabilitator can review such data either in MR or on his/her device later on to follow, analyse and adjust the treatment process if needed.

## Description

### Field of the Invention

The present invention relates to apparatus, methods, and/or computer programs for computer-implemented authoring and/or performing physical rehabilitation exercises in mixed reality.

### Background

Physical rehabilitation is a common treatment method to gradually and sustainably help patients regain their mobility functions, especially after suffering injuries, diseases or strokes, by applying various techniques such as stretching or performing exercises. Rehabilitation exercises often require patients to perform certain movements repeatedly to stimulate the capabilities and functions of muscles, joints, tendons or bones of the body parts that need to be treated. However, although many of those exercises are quite simple, they usually need a rehabilitator to entirely accompany the patient during practice sessions. For example, to improve legs flexibility of athlete patients, an exercise is to have a rehabilitator distributing a ball in different directions and requires a patient to move accordingly to kick the ball back to the rehabilitator. With many exercises, although rehabilitators are not required to participate in the practicing process with patients, they still need to be present to monitor and ensure the patients will perform the exercise correctly. For example, exercises which require patients to repeatedly move their hands, legs or heads from a position to another position might look simple but often need a rehabilitator to be present to check if the range of every movement of the patient is always correct as such repeated exercises might be boring but still painful, which demotivate and reduce the patient's concentration after a while. Likewise, sometimes the rehabilitator needs to be present to keep and adjust the pace of the exercise (make it faster or slower). As there are often more patients than rehabilitators, this will lead to a long list of patients waiting to be treated, which might sometimes make their health issues more severe due to late treatment.

Aside from the above, even when a rehabilitator can accompany a patient in the entire practicing sessions, there are also a number of drawbacks in this conventional setting. First, even the rehabilitator can still suffer fatigue and lose his concentration in monitoring and correcting the exercise performance of the patient after a while (e.g. imprecisely guiding the patient to move their body part to the correct position as set in the beginning or count inconsistent paces than expected). Second, with this conventional setting, the exercise performance of the patient is not completely and precisely recorded. Instead, the rehabilitator often just keeps track of some overall estimated numbers such as the average range of the patient's certain movements or how many reps the patient performed a certain exercise. Details such as if many times the patient's movements match the desired range or if not matched, what the movement deviation is, are difficult to record, which will affect the preview, the analysis and the planning of the patient's treatment process.

### Summary of the Invention

Aspects of the present invention are set out in the accompanying claims.

Embodiments of the invention disclosure may include apparatus, methods and computer programs which implement a mixed reality (MR) environment to allow rehabilitators to create interactive rehabilitation exercises by placing virtual objects with configurable attributes of interactivity in a space. In this context, an MR environment is one in which a real-world view is augmented with one or more virtual objects with which a user can interact by means of actions in the real world.

The virtual objects serve as markers or anchor points with which patients need to interact using certain body parts in order to perform the intended exercises which contribute to their rehabilitation process. Depending on their configured attributes, the virtual objects may react in response to the movements and the interactions of the patients (e.g., visually or auditorily simulate vibrations, movements etc.) to improve user perception of their performance. Once an exercise has been recorded in MR, a patient can continuously perform it at a precise manner without the rehabilitator being present. The rehabilitator can monitor the exercise performance remotely on an interface running on a remote monitoring device (e.g. PC, laptop or tablet). Moreover, the rehabilitator can also modify certain configurations of the exercise directly on the interface on the remote monitoring device, thus being able to remotely adjust the rehabilitation exercise to adapt to the patient. The exercise performance of the patient may be recorded in full or in part, and certain statistical indicators of the exercise performance may also be automatically computed. The rehabilitator can review the recorded exercise performance and associated statistics in order to analyse and perform necessary adjustments in the rehabilitation process.

Hence, embodiments of the invention may allow rehabilitators to create different rehabilitation exercises without requiring specific physical equipment such as balls, cones etc. which may be conventionally required.

Embodiments of the invention may allow patients to perform rehabilitation exercises at a high precision without one-to-one monitoring by a rehabilitator.

Embodiments of the invention may allow one rehabilitator to monitor remotely several patients' performance at a time.

Embodiments of the invention may allow a rehabilitator to adjust or modify a rehabilitation exercise programme remotely without having to physically go to the rehabilitation space of the patient.

Embodiments of the invention may enable a rehabilitator to review and analyse the performance of the patient through the patient's interactions with the virtual anchor objects as well as recorded joint movements, which may be represented in a wireframe or skeleton view.

### Brief Description of the Drawings

There now follows, by way of example only, a detailed description of embodiments of the present invention, with references to the figures identified below.
Figure 1 is a schematic diagram of apparatus for authoring and/or facilitating patients to perform rehabilitation exercises, in an embodiment of the invention.
Figure 2 is a schematic diagram of a computer system of the apparatus of Figure 1.
Figure 3 is a flowchart illustrating a computer-implemented method of authoring a rehabilitation exercise.
Figure 4 illustrates use of the apparatus in an authoring method when the rehabilitator is not wearing an MR headset, and virtual objects placed in the space can be seen on the physical display screen.
Figure 5 illustrates use of the apparatus in an authoring method when the rehabilitator is wearing an MR headset, and virtual objects placed in the space can be seen both on the physical display screen and via the MR headset.
Figure 6 illustrates a measuring feature of the authoring method which allows the rehabilitator to see the distance from a virtual object to another virtual object or to the physical display screen. Here the distances can be seen as augmentation on the physical screen.
Figure 7 illustrates the measuring feature as seen through the MR headset.
Figure 8 shows a configuration menu for configuration of a virtual object's attributes, as seen through the MR headset.
Figure 9 shows the configuration menu for specifying the body part required to interact with the virtual object.
Figure 10 is a flowchart illustrating a computer-implemented method of monitoring performance of a rehabilitation exercise by a patient.
Figures 11a and 11b show use of the apparatus by a patient forming a rehabilitation exercise respectively with and without an MR headset.
Figures 12a and 12b show use of the apparatus by a patient performing an exercise which requires him/her to move and kick virtual balls, respectively with and without wearing an MR headset.
Figure 13 shows a user interface which allows a rehabilitator to monitor multiple rehabilitation spaces on a monitoring device.
Figure 14 shows a user interface which allows a rehabilitator to view detailed performance of a specific patient.
Figure 15 shows the user interface of Figure 14 with a patient's wireframe or `skeleton' overlaid.
Figure 16 shows a user interface allowing the rehabilitator to remotely adjust a virtual objects' attributes to re-configure a rehabilitation exercise.
Figure 17 shows a patient at the rehabilitation space viewing on a display screen the remote adjusting/reconfiguring of virtual objects.
Figure 18 shows a patient viewing on an MR headset the remote adjustments/reconfigurations of virtual objects.
Figure 19 shows the rehabilitator reviewing the patient's performance on his personal device, using a skeleton mode to more accurately inspect joint movements.
Figure 20 shows the rehabilitator using an MR headset to review the patient's performance.
Figure 21 is a diagram of an example of a computing device on which one or more of the functions of the embodiments may be implemented.

### Description of Embodiments

### Apparatus

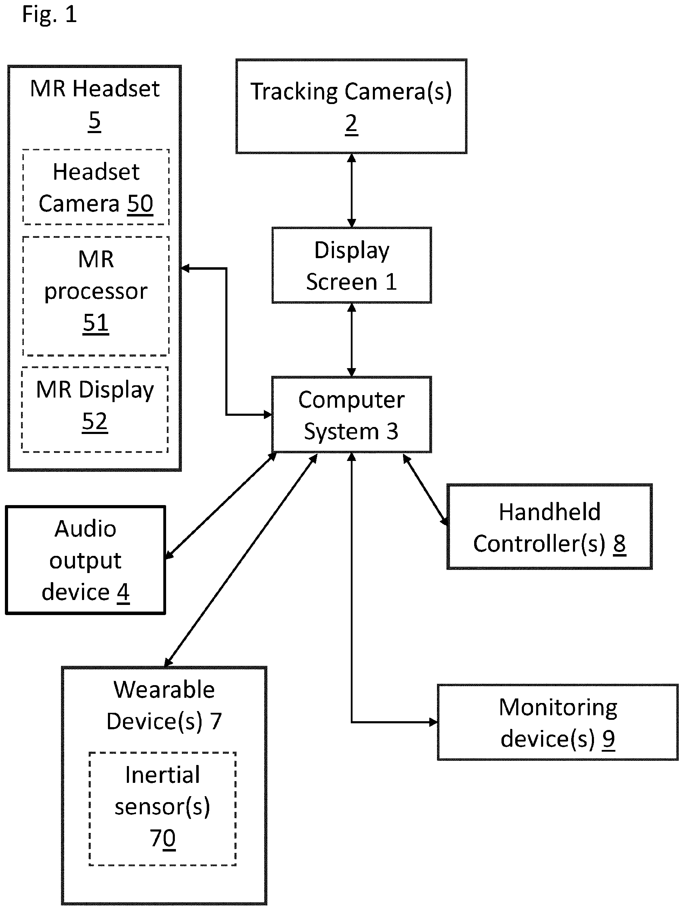

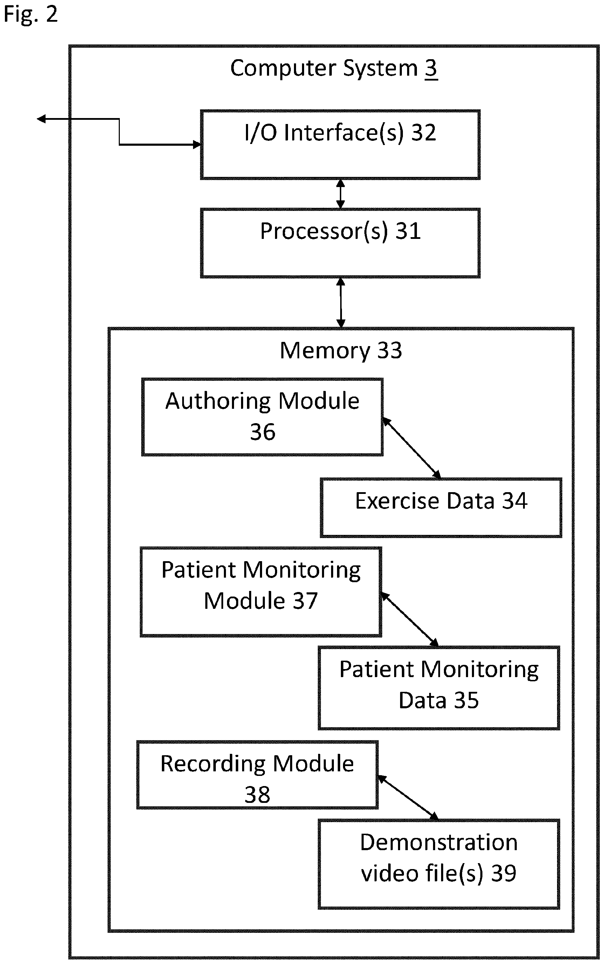

Figure 1 shows apparatus for authoring rehabilitation exercises and/or facilitating patients to perform authored rehabilitation exercises, in an embodiment of the invention. The apparatus comprises a computer system 3, shown in more detail in Figure 2. The computer system comprises one or more processors 31 which execute computer program code in order to record, in memory 33, rehabilitation exercise data 34 authored by an instructor or rehabilitator by means of program code comprising an authoring module 36, and/or to monitor performance by patients of rehabilitation exercises in accordance with the rehabilitation exercise data, for example by recording patient monitoring data 35 by means of program code comprising a patient monitoring module 37.

The computer system 3 includes one or more input/output interfaces 32, such as wired and/or wireless interfaces, for connection to and communication with one or more external devices as described below.

The apparatus includes at least one physical display screen 1 at a location where patients perform rehabilitation exercises and/or an instructor or rehabilitator authors rehabilitation exercises. At least one optical camera 2, which will be later referred to as global tracking camera(s), is connected to the display screen(s) 1 and the computer system 3 and is arranged to capture images within a space in which authoring or performing rehabilitation exercises take place,. The tracking camera(s) 2 may have both colour-sensing and depth-sensing capabilities in order to capture not only colours but also depth information of humans and objects in the space. The rehabilitation space may be defined as a 3D volume within which the tracking camera(s) can track real objects.

Images captured by the tracking camera(s) 2 may be shown on the display screen 1 in a mirror-like manner, together with virtual augmentation images generated by the computer system 3 and provided as an overlay or modification of the camera images to provide mixed-reality assistance to the patients.

The patients and/or the rehabilitator may optionally wear a mixed reality (MR) headset 5 which gives the wearer a 3D view of the real world (as seen through the headset or provided as a video feed to a display 52 in the headset 5) with an overlay of one or more virtual objects with which the wearer can interact in augmented 3D space. In this case, the virtual object(s) may comprise one or more spatial anchors at predefined positions in the physical space. Thanks to this, the patient and the rehabilitator may have two ways to visually perceive virtual objects 12 for assisting exercise performance: via the MR headset 5 (if they wear it) or directly on the display screen 1 without the need of an MR headset 5, as mentioned previously.

The MR headset 5 may have a depth-sensing headset camera 50 mounted thereon to capture the environment around the wearer. The MR headset 5 may also be wirelessly connected to the computer system 3. The position and orientation of the MR headset 5 worn in the rehabilitation space may be tracked by the global tracking camera(s) 2. An approach to do this is to attach distinctive indicia such as a marker/visual pattern on the MR headset 5, which is then tracked based on both colour/image and depth information from the global tracking camera(s) 2.

The MR headset 5 may also include an MR processor 51 which generates one or more virtual object images, based on virtual object data provided by the computer system 3, for combination with a live feed from the headset camera 50 to generate an MR video feed on the MR display 52. The appearance of the virtual object may be determined according to the detected position and orientation of the MR headset 5, so that the virtual object appears as an object in the space to the wearer of the MR headset. Alternatively, processing of the virtual object data to generate a virtual object image may be performed by the computer system 3, although this is less preferred due to potential lag in the wireless connection between the MR headset 5 and the computer system 3.

Optionally, the rehabilitator may use a handheld controller 8 as a position indicator to position virtual objects in the space during the exercise authoring stage. The handheld controller 8 may be wirelessly connected to the computer system 3.

The handheld controller 8 may consist of three main components:
- indicia such as one or more physical, visual patterns, for example on a head portion of the handheld controller 8, that allow the handheld controller 8 to be recognized and tracked by the global tracking camera 2.
- one or more input devices, such as physical buttons or switches, which allow the rehabilitator to provide user input as commands to the computer system 3 to place, select or delete a virtual object at a position indicated by the handheld controller 8.
- a physical feedback unit for providing feedback to the user, such as light, sound or vibration to confirm to the rehabilitator when a command has been sent to, or received by the computer system 3.

Optionally, the apparatus also includes one or more wearable devices 7. The wearable device(s) 7 may include a haptic feedback unit to provide feedback to the wearer. Each wearable device 7 may have indicia such as a distinctive visual pattern which allows it to be tracked by the global tracking camera 2. Each wearable device 7 may also have one or more inertial sensors 70 such as an accelerometer or gyroscope, to improve position and orientation tracking of the user, which may be used by the computer system 3 in combination with the optical tracking camera system. Each haptic device 7 may be wirelessly connected to the computer system 3. Each haptic device 7 may be programmed to generate haptic feedback (e.g., vibration) to the user.

The computer system 3 may be connected to one or more monitoring devices 9, for example through a wired or wireless network connection or by a local wireless connection such as a BlueTooth (RTM) connection. The monitoring device(s) 9 may be personal computing devices such as a desktop, laptop, tablet or smartphone which may store and execute software, such as an app to allow the rehabilitator to monitor, adjust, review and analyse the patient's exercise performance.

### Authoring rehabilitation exercises

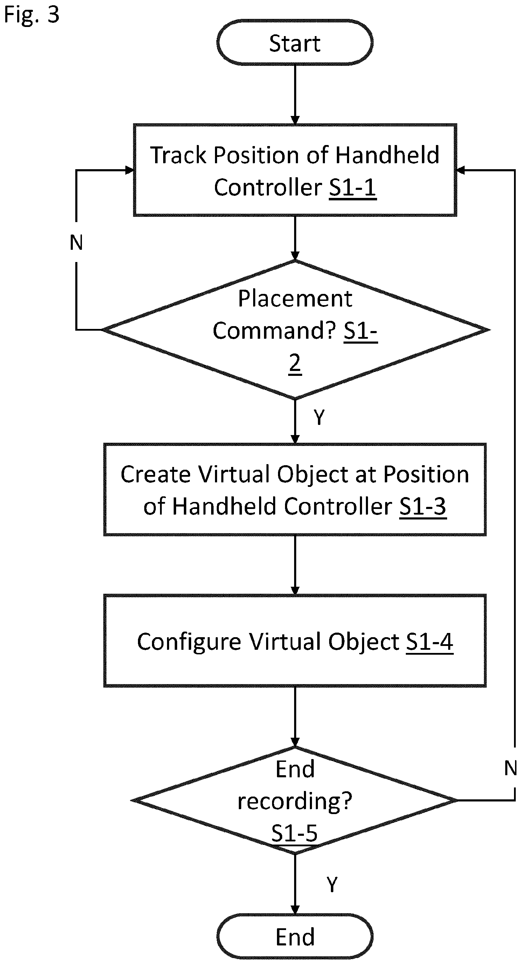

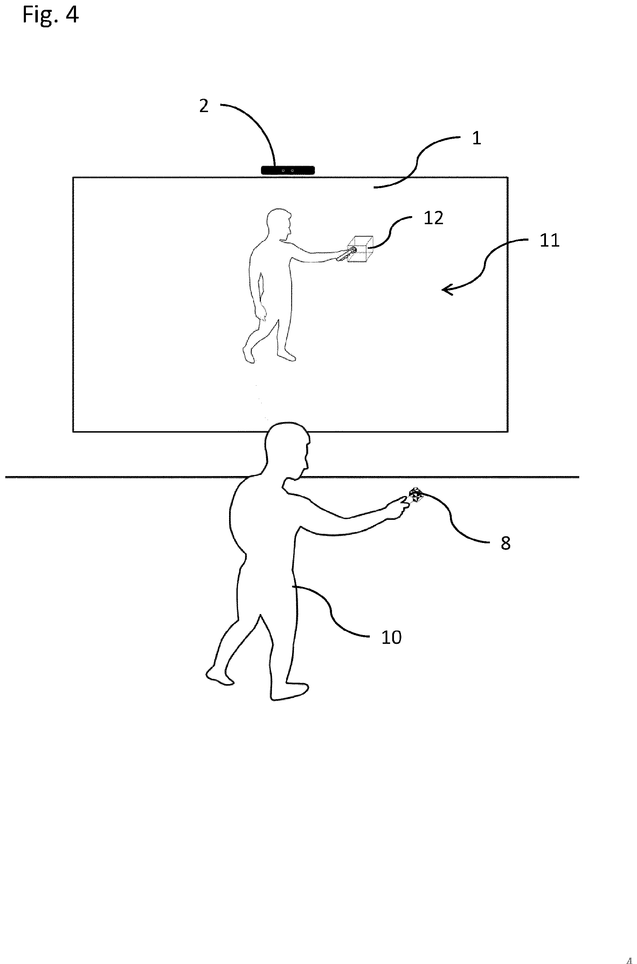

To author a rehabilitation exercise, a rehabilitator uses the apparatus as described above to place one or more virtual objects in the rehabilitation space, which virtual objects serve as interaction anchors for patients to align their body parts to while performing the exercise. An embodiment of a computer-implemented method of authoring a rehabilitation exercise will now be described with reference to the flowchart of Figure 3 and the diagrams of Figures 4-9.

First, the rehabilitator 10 starts the authoring procedure by providing a predetermined user input to the computer system 3, for example by pressing a designated start button on the handheld controller 8. The computer system 3 then launches the authoring module 36 to initiate a recording process to create an exercise record comprising data representing one or more virtual objects with corresponding configuration data, as described below.

During the recording process, the computer system 3 tracks (step S1-1) the position of the handheld controller 8 using the global tracking camera(s) 2. This tracking may be done continuously or in response to user input, for example as described below.

In order to place a virtual object 12 at a position in the rehabilitation space, if using the handheld controller 8, the rehabilitator aligns the handheld controller 8 at that position and provides a placement command as a user input (step S1-2), for example by caused by the user pressing a corresponding physical button on the handheld controller 8. The position of the handheld controller 8 at the position specified by the placement command is detected or estimated by the global tracking camera(s) 2, and stored as a position of the virtual object.

An exemplary approach to estimate the position of the handheld controller 8, which carries a visual pattern, will now be described. The computer system 3 may utilize two image sources from the global tracking camera(s) 2: colour images and depth images. In colour images, the computer system 3 locates the 2D position of the head of the handheld controller 8 by using computer vision (e.g. using a machine learning model) to detect the distinctive visual patterns on the head of the handheld controller 8. When the 2D position of the handheld controller 8 is determined, the corresponding depth images are also determined. As the colour/pattern images and the depth images are both acquired from the same global tracking camera(s) 2, the disparity of the content captured in them is minor. Thus, by matching the 2D position detected in the colour images with that in the depth images (which are normally grey scale), the computer system 3 determines the position of the handheld controller 8 in the depth images. After that, based on the depth information encoded in the depth images, the computer system 3 estimates the 3D position of the handheld controller 8 in the rehabilitation space.

In another approach, the indicia on the handheld controller 8 comprise infrared light-emitting dots which are captured by the global tracking camera(s) 2. Triangulation between the dots may then be employed to estimate the position of the handheld controller 8 in the space.

In response to the rehabilitator confirming a placement command by pressing the corresponding physical button on the handheld controller 8, the computer system 3 creates a virtual object (S1-3) (e.g. a 3D sphere, a 3D cube, a 3D bar, etc.) at the position of the handheld controller 8 in the rehabilitation space at the moment the command was made. The computer system 3 adds the virtual object as virtual object data to the rehabilitation exercise data 34 for the currently authored exercise record.

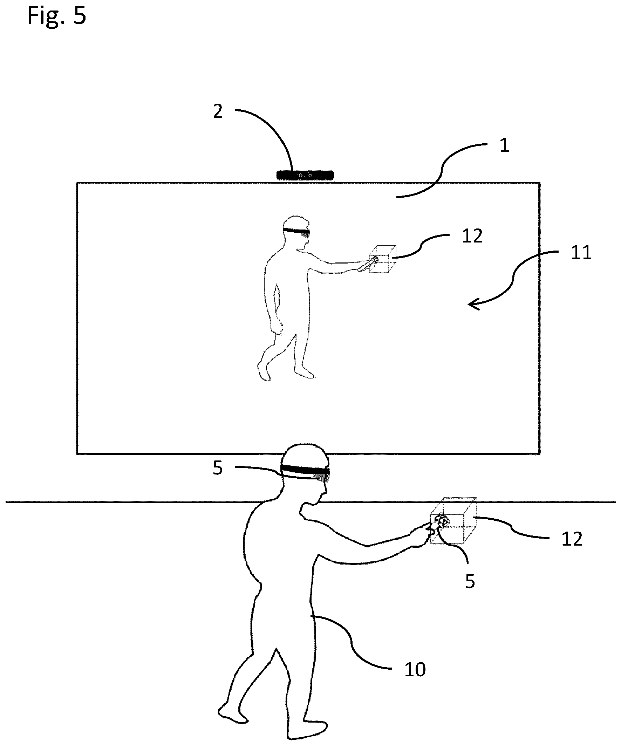

The computer system 3 may display the created virtual object 12 to the rehabilitator in the rehabilitation space in either one or both of two ways: on the display screen 1 and on the MR headset 5. In the first way, as shown in Figure 4, the rehabilitator 10 can simply look at the display screen 1 in the rehabilitation space. The display screen 1 shows the colour video feed(s) 11 streamed directly from the global tracking camera(s) 2. Once a virtual object 12 is added into the space, it will appear at the corresponding position as a virtual object image in the displayed video feeds 11. Depth information of objects and people in the space, captured by the global tracking camera(s) 2, may also be used by the computer system 3 and/or the MR processor 51 to create necessary occlusion in order to provide realistic perception of the position of the virtual object in the physical space. This can be seen either with (Figure 5) or without (Figure 4) an MR headset.

The second way is for the rehabilitator 10 to view the space through the MR headset 5. Once a virtual object 12 is placed in the rehabilitation space, this information is sent by the computer system 3 to the MR headset 5. The MR headset 5 also receives information about its position and orientation in the rehabilitation space from the computer system 3, based on tracking by the global tracking camera(s) 2. Based on this, the MR processor 51 calculates the position and orientation of the virtual object 12 relative to it and displays the virtual object image in an MR space centred around the MR headset 5. With this second way, as shown in Figure 5, the rehabilitator 10 can still see an added virtual object 12 in the space even when not looking at the screen 1.

The rehabilitator 10 may also grab the virtual object 12 and shift it to another position or delete an existing virtual object 12 via a predetermined user input such as pressing the corresponding physical buttons on the handheld controller 8, or by a gesture as described below.

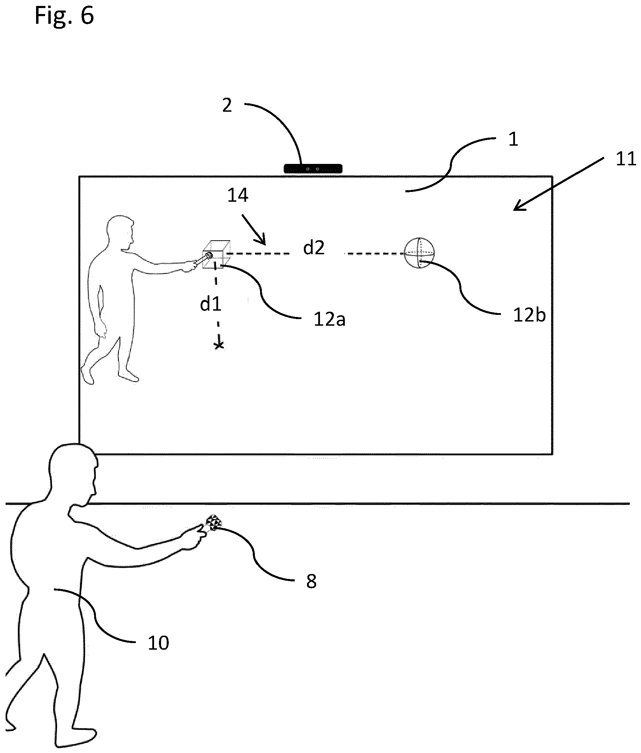

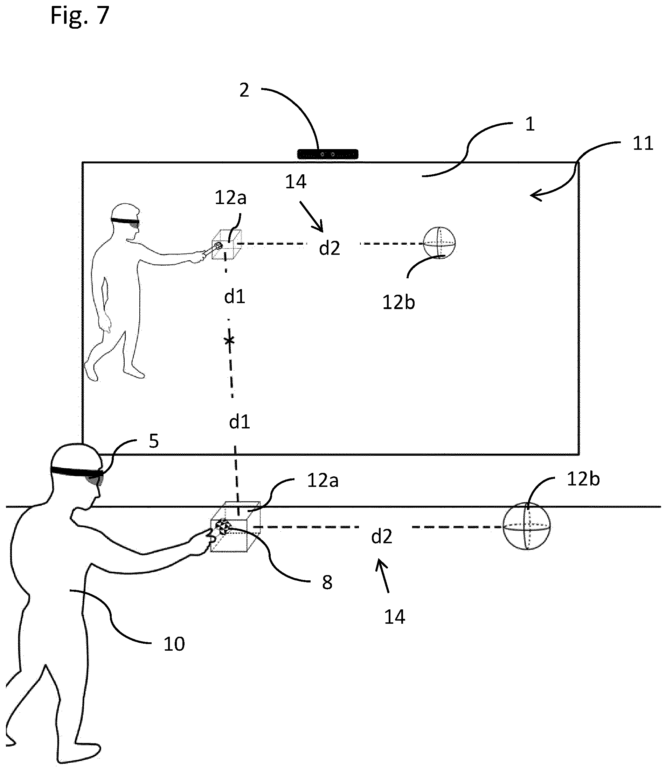

To support the rehabilitator in precisely positioning virtual objects in the space, the computer system 3 may provide the rehabilitator with a virtual measuring tool 14, which can be seen on the physical screen as shown in Figure 6 or through the MR headset 5 (if the rehabilitator 10 is wearing one) as shown in Figure 7. This virtual measurement tool 14 allows the rehabilitator 10 to see the distance d1 between a first virtual object 12a to the physical display screen 1 and/or the distance d2 between a first virtual object 12a to a second virtual object 12b. The distances can be estimated from the depth information acquired by the global tracking camera(s) 2.

If the handheld controller 8 cannot be detected, (for example if the handheld controller 8 is occluded or falls outside of the field of view of the global tracking camera(s) 2), the computer system 3 may provide an alert (e.g., visual via display 1 or auditory via audio output device 4) to inform the rehabilitator 10 about the error so that he/she can move the handheld controller 8 such that it can be detected by the tracking camera(s) 2.

Alternatively, the headset camera(s) 50 may be capable of capturing and recognizing the wearer's hand gestures, for example by means of one or more front-facing depth cameras. In that case, the external handheld controller 8 may not be necessary and the rehabilitator 10 may instead perform virtual object placement using one or more predetermined hand gestures (e.g., pinching). In this case, the position of the virtual object 12 in the rehabilitation space is determined in a reversed direction compared to the previous approach. The position of the virtual object 12 is first determined in relation to the position and orientation of the MR headset 5, based on the tracking system of the MR headset 5. Then the position and orientation of the virtual object 12 in the rehabilitation space is interpolated from its relative position and orientation to the MR headset 5 and the position and orientation of the MR headset 5 in the rehabilitation space, as tracked by the global tracking camera(s) 2.

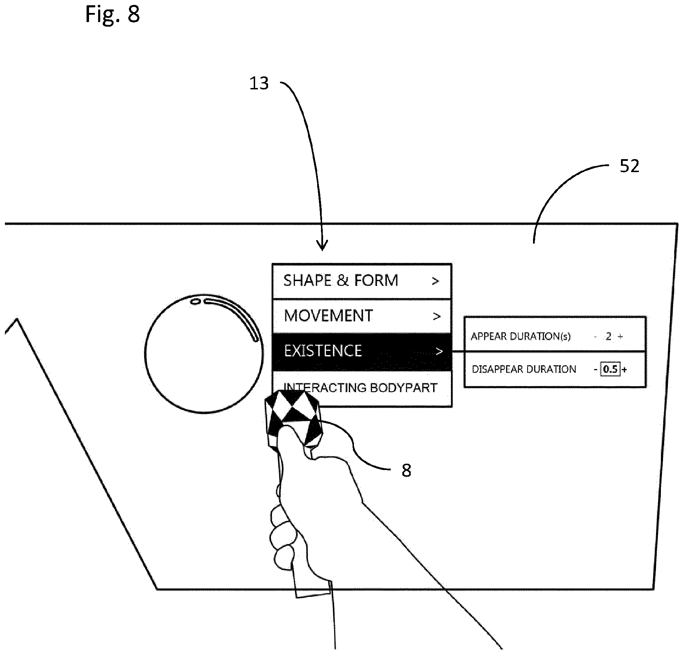

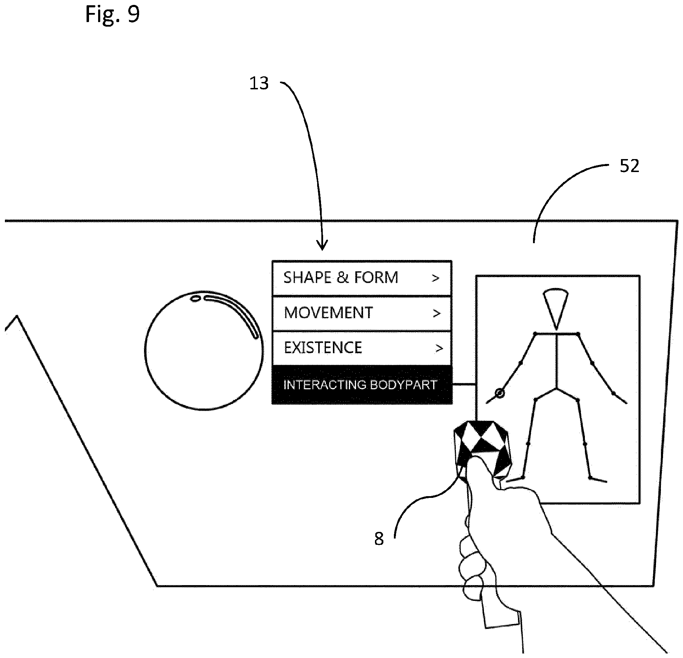

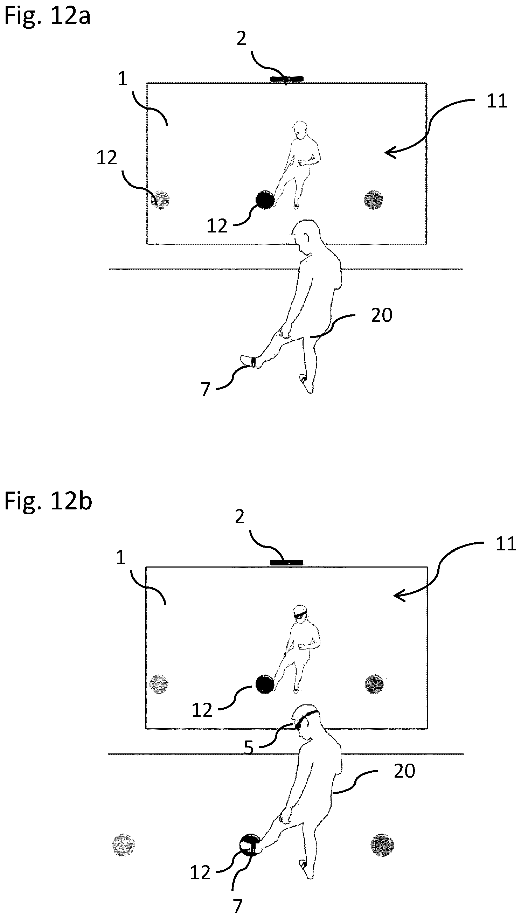

Once a virtual object 12 is placed, the rehabilitator 10 may configure (S1-4) the virtual object 12 using an interactive configuration menu 13 displayed on the display screen 1 and/or in the MR display 52 as shown in Figures 8 and 9. The configuration may relate to the appearance of the virtual object 12 such as shape (e.g., sphere, cube, bar box, pyramid etc.), colour and/or size. The rehabilitator 10 may also configure different interactive attributes of the virtual object 12 according to how it will be used in a rehabilitation exercise. For example, if the rehabilitation exercise is to require a patient to continuously and rapidly move in the rehabilitation space to kick a virtual ball generated as a virtual object 12 within the rehabilitation space by the computer system 3, then the rehabilitator 10 may first place a virtual object 12 with the appearance of a sphere representing the ball at an initial position within the space, then indicate different positions that the ball should move to as well as the speed of the movement, using a Movement option in the interactive menu 13. An example of the use of this exercise by a patient is described below in relation to Figures 12a and 12b.

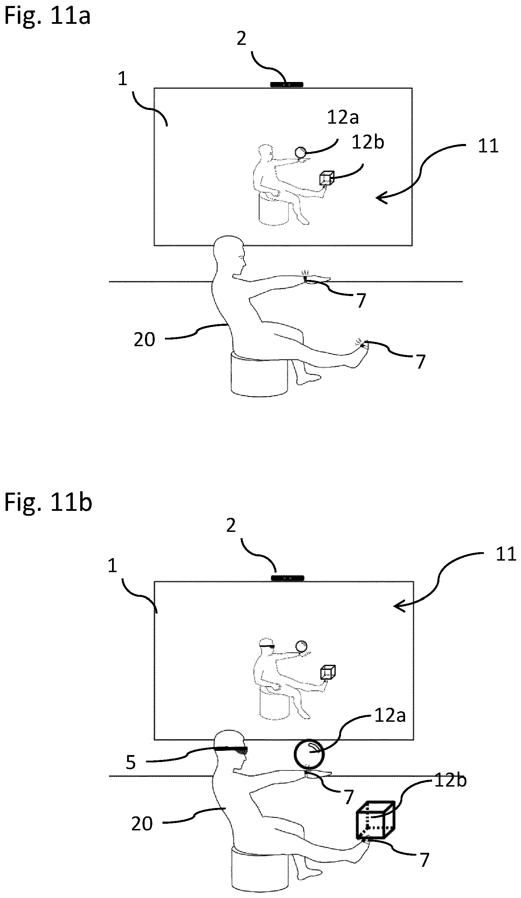

Another example of an exercise which may be authored by the rehabilitator 10 is an exercise where the patient will need to raise and move his/her hand and/or foot to tap different positions in the air, to help recover their shoulder, hand, leg or foot functions, as shown in Figures 11a and 11b which show the patient 20 performing a rehabilitation exercise respectively with and without an MR headset 5. In this example, corresponding haptic devices 7 worn on the patient's wrist and foot indicate when these reach the respective virtual objects 12a, 12b.

In creating such an exercise in mixed reality, the rehabilitator 10 may sequentially create virtual objects 12a..n with the appearance of spheres or cubes at different defined positions within the space. Then for each created virtual object 12, the rehabilitator 10 may configure, using an Existence option on the interactive menu 13 as shown in Figure 8, how many times each virtual object 12 should appear, the duration of each appearance and the interval between two consecutive experiences, depending on the physical situation of the patient and the rehabilitation progress.

Furthermore, the rehabilitator 10 may configure which body part of the patient is supposed to interact with the virtual object 12, using an Interacting Body Part option in the interactive menu 13 as shown in Figure 9. When the patient 20 performs the exercise, if this information is available, the computer system 3 will use the body part configuration information for tracking the interaction of the correspondent body part of the patient with the virtual object 12.

### Recording exercise performance demonstration

Once the virtual objects 12 involved in assisting the rehabilitation exercise has been recorded as exercise data 34 by the computer system 3, the rehabilitator 10 may additionally record an associated video file or files containing a demonstration to patients of how to perform the exercise, for example using a recording module 38 which uses the global tracking camera 2 to record demonstration video file(s) 39 in the memory 33 of the computer system 3. The rehabilitator 10 may start at the position where the patient is supposed to be at the start of the exercise, launch the recording module 38 on the computer system 3 and start performing the exercise by moving his/her body parts so as to interact with the virtual objects 12 as required by the authored exercise data 34 representing the exercise (e.g., legs move up until the knees touch the virtual objects 12, bending neck backwards until the back of the head touches a sphere, etc.). The entire exercise performance of the rehabilitator 10 together with the appearance of the virtual objects 12 is recorded as the demonstration video file(s) 39 by the computer system 3. Furthermore, the movements of the rehabilitator's joints may also be recorded as a separate but associated movement file, for example as a set or sequence of 3D coordinates in the rehabilitation space, with associated joint IDs.

The recorded demonstration video file(s) 39 and/or movement file(s) can be played back later to demonstrate how to perform the exercises to the patient. For example, while the demonstration video file(s) 39 can be played directly on the physical display screen 1 or on a virtual display seen through the MR headset 5, the movement file(s) may be displayed through the MR headset 5, with the 3D coordinate data of the joint movements generated as virtual images and played back at the position where the rehabilitator's joints were positioned when performing the demonstration.

### Performing rehabilitation exercises

After the rehabilitation exercise has already been setup by recording exercise data 34 of an exercise record by the authoring module 36 in the computer system 3, a patient can perform the exercise by interacting with the virtual objects 12 as defined in the exercise data 34 following the instruction of the rehabilitator 10, either via in-person demonstration or using the pre-recorded performance.

The patient 20 may use the same or similar apparatus to that used by the rehabilitator 10, as shown in Figure 3. Possible differences between the apparatus used by the patient 20 and the rehabilitator 10 are discussed below. Where the rehabilitator 10 and the patient 20 use different apparatus having a different instance of computer system 3, the exercise data 34 may be transferred from the rehabilitator's computer system 3 to the patient's computer system 3 using any conventional file transfer technique.

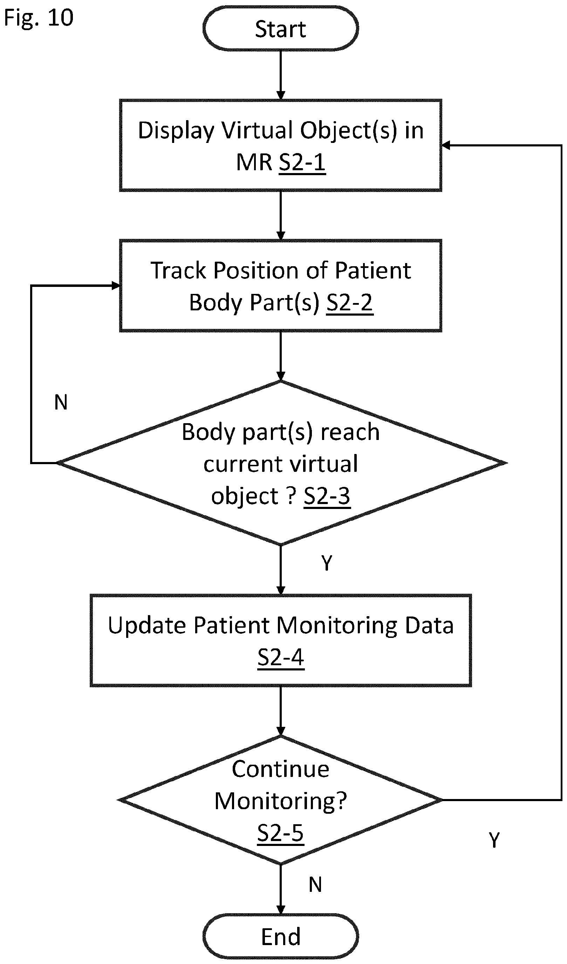

The computer system 3 monitors the performance of the patient 20 in comparison with the sequence of predefined virtual objects 12 in the exercise data 34, in a method as shown for example in Figure 10. The computer system 3 displays the predefined virtual object(s) 12 to the patient in mixed reality (step S2-1), via the display screen 1 and/or the MR headset 5. The computer system 3 tracks the movement of the patient's body part(s) (step S2-2), for example using the tracking camera(s) 2 and/or one or more wearable devices 7 worn by the patient 20. When the computer system 3 detects that the requisite body part reaches the required virtual object 12 defined by the exercise data 34 (step S2-3), the computer system 3 may provide feedback to the patient 20. The computer system 3 then updates the patient monitoring data (step S2-4), and may perform any consequential action defined in the configuration data of the virtual object 12. The computer system 3 may continue monitoring (step S2-5) until an end condition is reached.

Some exercises may require the patient to repeatedly reach the same virtual object 12, in which case the computer system 3 may update the patient monitoring data 35 by counting the number of repetitions (`reps') of the patient reaching that virtual object 12. Alternatively, or additionally, where an exercise requires the patient to hold a position for a predetermined length of time, the virtual object 12 may only be registered as having been reached in step S2-3 if the required part of the patient's body is held in the position of the virtual object 12 for a predetermined length of time.

If there are no more virtual objects 12 in the sequence or some other end condition is met such as a time-out, the monitoring process ends and the patient's performance against the exercise defined by the exercise data is recorded as patient monitoring data 35.

The patient 20 may wear one or more devices 7 which render haptic feedback to the patient 20 according to the interaction of one or more corresponding body parts of the patient with one or more virtual objects 12 defined by the exercise data 34. This may help the patient 20 be better aware of their movements and interaction with the virtual object(s) 12 to perform the exercise correctly.

For example, in a rehabilitation exercise, the patient 20 may be required to move and kick virtual balls 12a-n placed at different positions in the space. When the patient 20 kicks a virtual ball 12, the wearable device 7 worn on the kicking leg will vibrate and the virtual ball 12 will move in the kicking direction to inform the patient 20 about the successful kick. The patient 20 can then move on to the next virtual object 12, for example kicking a new virtual ball 12. After a period configured by the rehabilitator 10 as an Existence configuration, as described above, the virtual ball 12 may appear again at the same position waiting for a new kicking turn of the patient 20.

In another example, the patient 20 may wear the wearable device 7 at the back of his/her ankle, which device 7 may generate vibration when the patient 20 moves his/her leg backward until touching a predefined virtual object 12. In this exercise, the virtual object 12 is placed behind the patient 20 so might not be easily observed by the patient; thus providing haptic feedback in this case will help effectively inform the patient 20 that their movement has reached the required range defined in the exercise data 34.

Alternatively, audio can also be optionally played via the audio output device 4 to help the patient 20 self-monitor their performance. For example, based on the patient's posture estimated from the position of body parts captured by the tracking camera(s) 2, the computer system 3 might recognize that in the current posture it might be difficult for the patient 20 to see rep counts on the display screen 1 (e.g., how many reps have been done over the total of reps to be completed), and thus may automatically generate the sound of the counting. Additionally or alternatively, the computer system 3 may generate pseudo haptic feedback using visual (e.g. via display 1) or auditory (e.g. via audio output device 4) stimuli (e.g., visually showing animation to illustrate positional or shape changes of the virtual object 12 when the patient's body parts "touch" it or play suitable sounds to inform the patient 20 about the contact) applied to the virtual object(s) 12 to inform the patient 20 about the interaction between his/her body parts and the virtual object(s) 12. Thus, the patient 20 may stay aware of the correctness of their posture and movement during the exercise performance. The virtual object(s) 12 may automatically appear and react to the patient's interaction according to their defined configurations, so that the patient 20 can correctly perform the exercise, in terms of posture, pace and rhythm, by interacting with the virtual object(s) 12, without the need of on-site presence of the rehabilitator 10.

The exercise performance of the patient is recorded by the computer system 3 as patient monitoring data 35. The recorded patient monitoring data 35 may include one or more video files of the patient 20 performing the exercise in the MR environment, with the video content showing the virtual objects 12, the 3D coordinates of the patient's joints across the rehabilitation time as well as the number of times each virtual object 12 was interacted with.

### Monitoring exercise performance

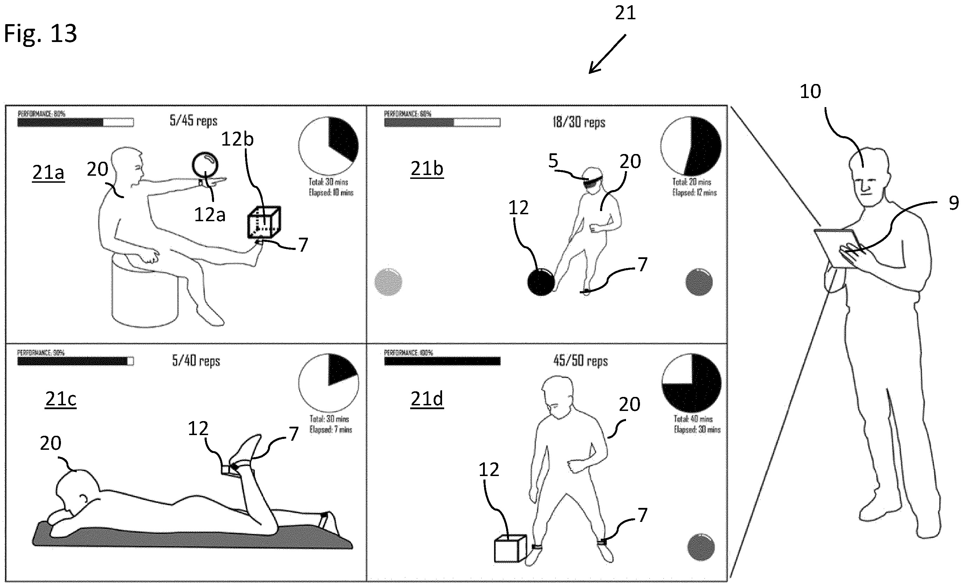

As the patient 20 can perform the exercise correctly by interacting with preconfigured virtual objects 12 without the need for the rehabilitator to be present on-site, the rehabilitator 10 can monitor the exercise performance of multiple patients 20, potentially at different locations, at a time, by means of the monitoring system 9 which has user interface software which can run on PC, laptop or mobile devices like a tablet or smartphone, allowing the rehabilitator 10 to remotely monitor the rehabilitation exercise performance of multiple patients 20 as captured by the global tracking camera(s) 2 in their respective space. As shown in Figure 13, the user interface 21 may consist of multiple windows 21a-d, respectively displaying video streams of the different rehabilitation spaces and the patients 20 within that the rehabilitator 10 needs to monitor. Each window may display the video stream from the global tracking camera(s) 2 with virtual objects 12 overlaid at their corresponding defined positions.

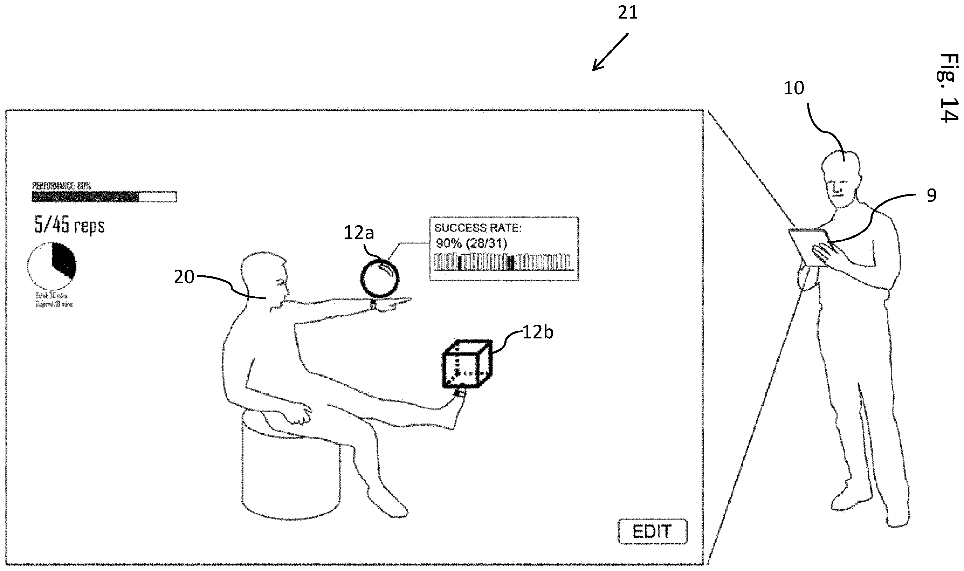

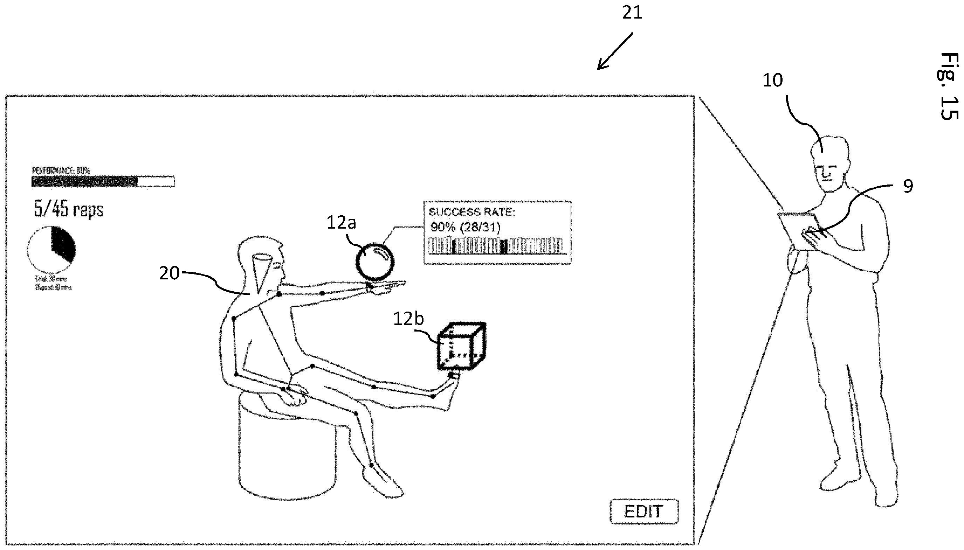

The rehabilitator 10 can choose to view more details of the rehabilitation space and the patient's performance in a selected window 21a-d. In viewing more details in a specific window 21a... d, the rehabilitator 10 can enable a "skeleton mode" which will display as a wire frame the joints of the patient 20 performing the exercise as captured by the global tracking camera(s) 2. The rehabilitator 10 can view the joints overlaid directly on the video stream as shown in Figure 15 or separately in a 3D view where the rehabilitator 10 can freely navigate around to inspect the patient 20 from different perspectives for effective diagnosis of the movements of the patient 20. The historical performance and movement data of the patient 20 since starting the exercise, as recorded in patient monitoring data 35, can also be played back on the user interface 21 to support the rehabilitator 10 to review the patient's performance during the time he/she was not attending, as shown in Figure 14.

The user interface 21 also allows the rehabilitator 10 to view interaction details of the virtual objects 12. For example, the rehabilitator 10 can choose to view how many times a specific virtual object 12 was interacted with by a certain body part of the patient 20. There are two approaches, described below, to determine if a virtual object 12 was successfully interacted with by a certain body part of the patient 20 as well as the patient's performance (i.e. the success rate of the body part touching the virtual object 12 over the total reps performed).

In one approach, if in the configuration of the virtual object 12 the rehabilitator 10 has explicitly indicated via the configuration menu 13 which body part is supposed to interact with the virtual object 12, the computer system 3 tracks the position of that body part. When the body part is moving and has reached a position where there is a change in its movement direction, that point is considered to be a turning point. The computer system 3 may consider the turning points where the body part's positions are nearby the target object as 'reps' or repetitions.

If the rehabilitator 10 has not explicitly indicated which body part is supposed to interact with the virtual object 12, the computer system 3 may perform a pre-processing step to identify the body part which was likely supposed to interact with the target virtual object 12. To do this, the computer system 3 identifies all the patient's joints tracked by the global tracking camera(s) 2. For each joint, the computer system 3 calculates the average/mean position of the joint throughout the entire performance period. Then the joint with the average/mean position closest to the target virtual object 12 will be identified as the body part of interest. After that, the computer system 3 will determine all the reps of the body part following the criteria mentioned above.

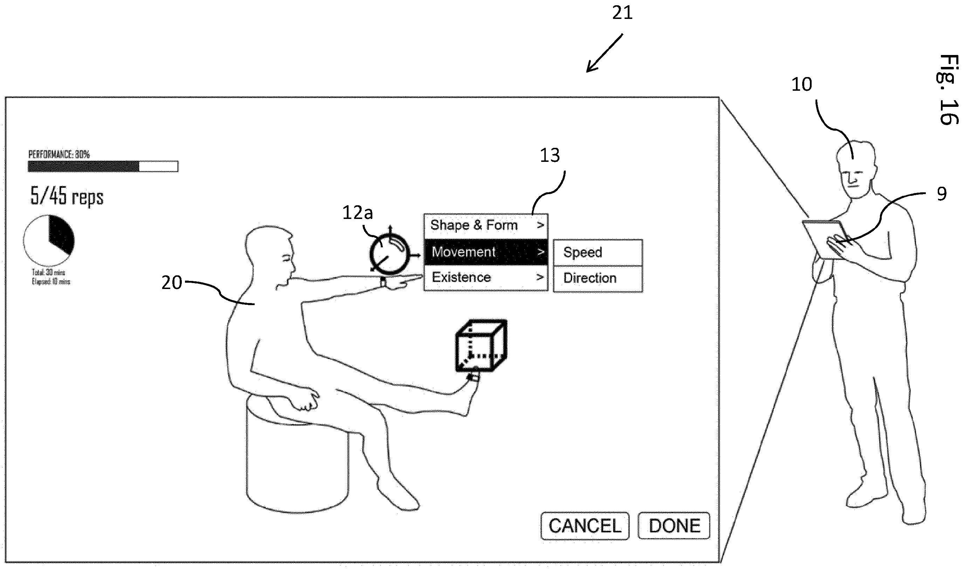

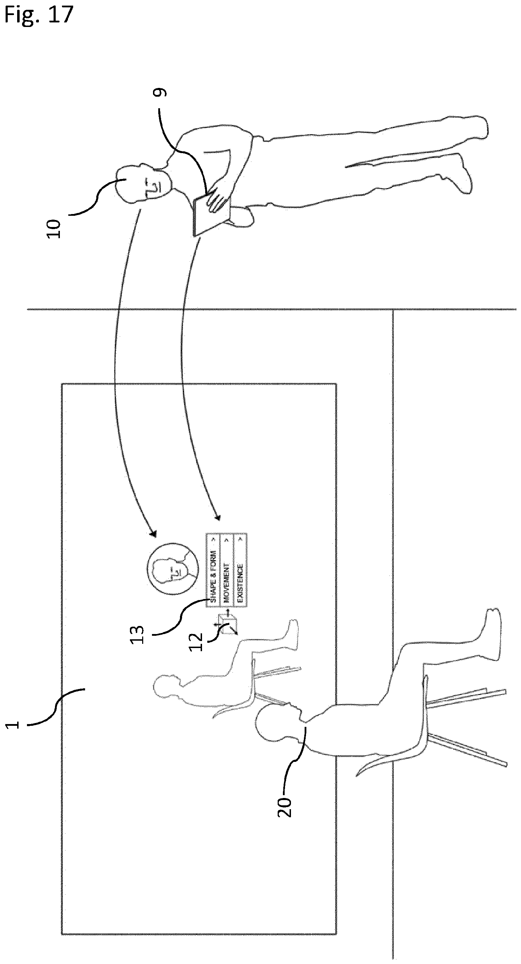

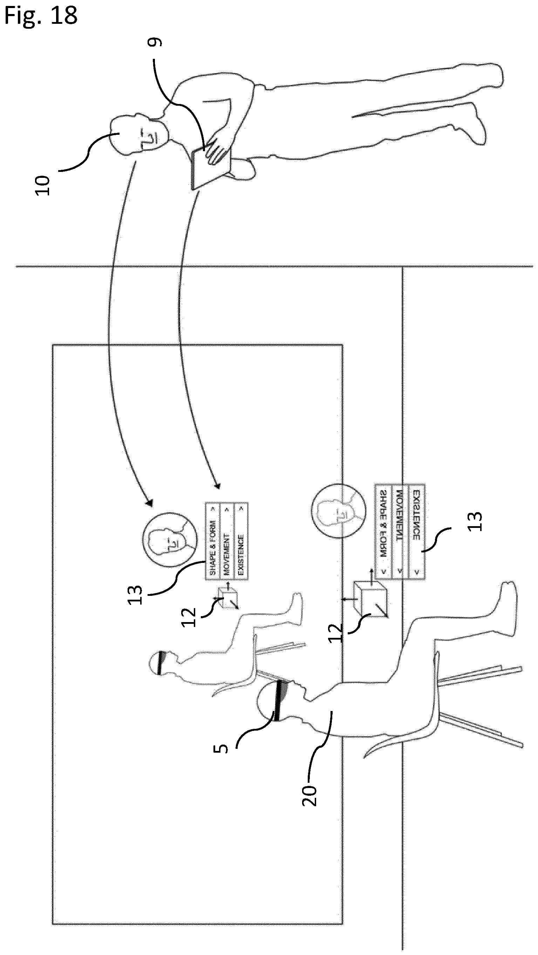

The rehabilitator 10 may also re-configure certain attributes of the virtual object(s) 12 directly on the user interface 21 to make the exercise better fit the patient's current physical status. For example, via this user interface 21 as shown in Figure 16, the rehabilitator 10 can adjust various attributes of a virtual object 12 such as position, duration of presence, appearance interval, movement speed, shape, colour, using a configuration menu 13 similar to that used during the authoring phase. The patient 20 may see the interaction and the representation of the rehabilitator 10 performing the adjustments/reconfigurations on the physical display screen 1 as shown in Figure 17 or directly in the rehabilitation space via the MR headset 5 as shown in Figure 18.

### Reviewing and analysing exercise performance

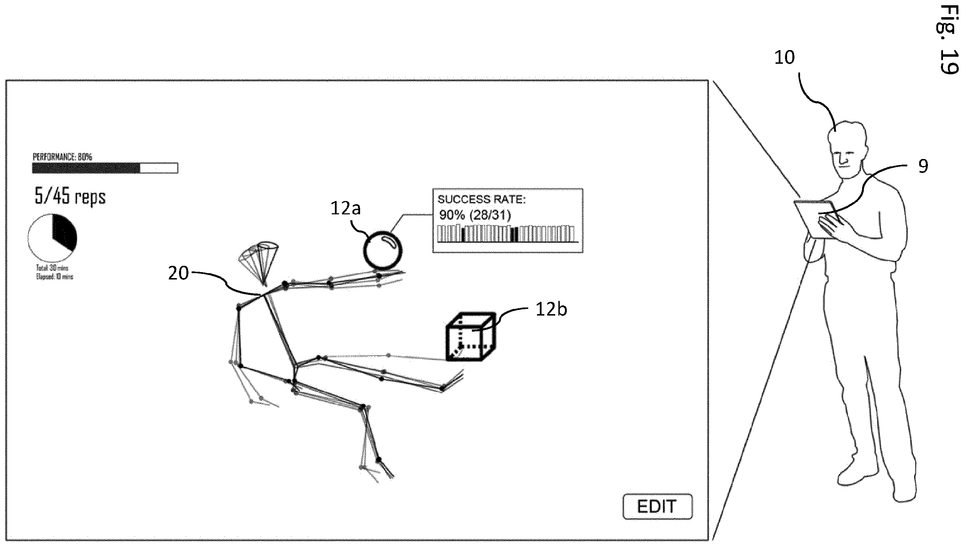

The patient's movements when performing the exercise are recorded as patient monitoring data 35 by the computer system 3 and can be played back afterwards on the rehabilitator's monitoring device 9. Similar to the monitoring interface 21, the rehabilitator 10 can view the patient's recorded exercise performance as video streams. Movements of the patient's joints can also be overlaid on the videos at their corresponding positions or the joints can also be viewed separately in a 3D interface, as shown in Figure 19. For each virtual object 12, the computer system 3 may automatically compute and display certain statistical indexes such as the mean position and the deviation between the positions of the corresponding body part to the virtual objects 12 during the entire exercise period. Historical movement data of the patient's joints in the space can also be visualized to provide the rehabilitator 10 an overview on the entire training period, as shown in Figure 19.

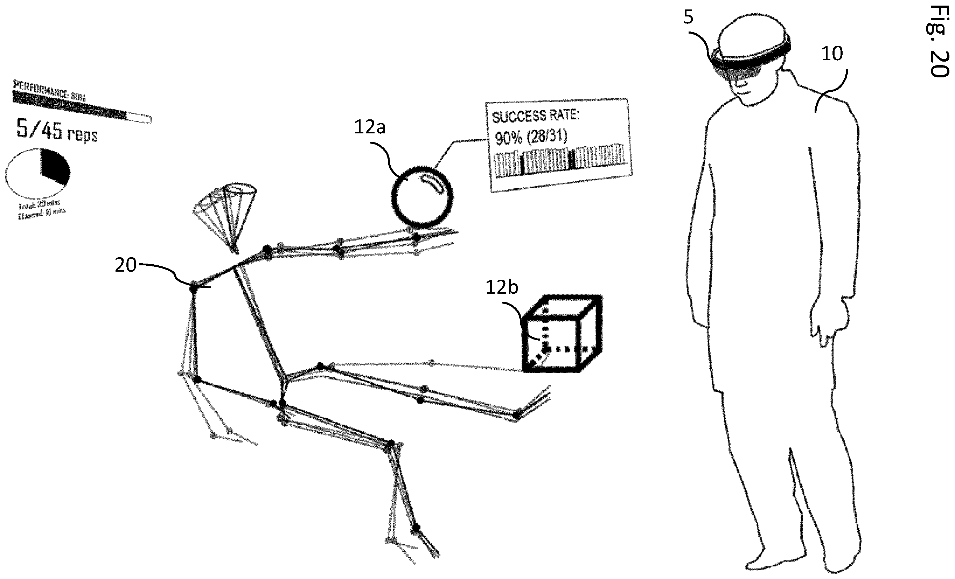

If the rehabilitator 10 is wearing MR headset 5, he can view the 3D coordinate data of the patient's joints rendered in 3D through the MR headset 5 at real size or a larger scale for accurate inspection and diagnosis, as shown in Figure 20. The rehabilitator 10 wearing the MR headset 5 can walk around the 3D rendering of the patients' movement data to observe the joints' historical positions from different perspectives and distances.

### Computer System

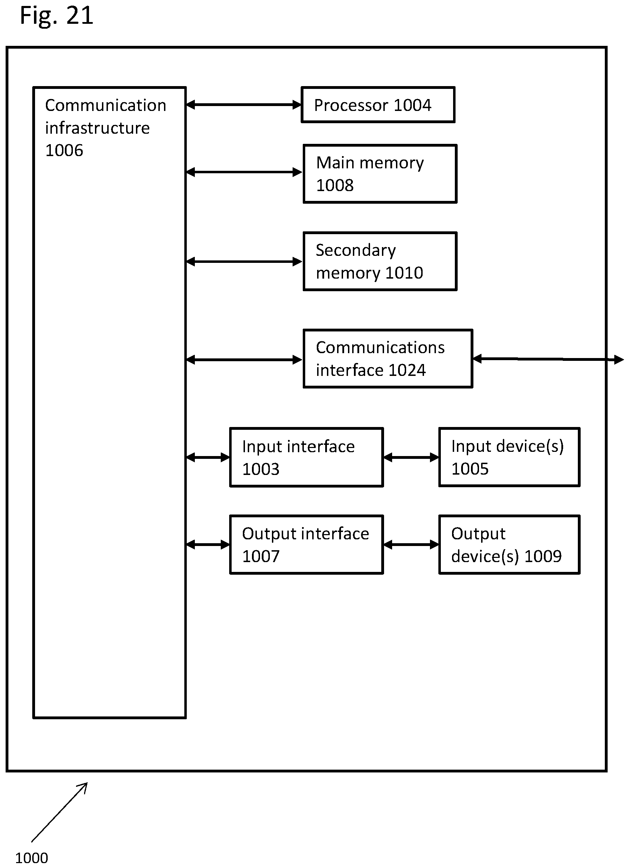

Figure 21 illustrates an example computer system 1000 in which embodiments of the present invention, or portions thereof, can be implemented as computer-readable code to program processing components of the computer system 1000. Various embodiments of the invention are described in terms of this example computer system 1000. For example, the computer system 3, MR headset 5 or monitoring device 9 can each be implemented as a computer system such as system 1000. The methods illustrated by the flowcharts of Figure 3 and Figure 10 can be implemented in system 1000. After reading this description, it will become apparent to a person skilled in the relevant art how to implement the invention using other computer systems and/or computer architectures.

Computer system 1000 includes one or more processors, such as processor 1004. Processor 1004 can be a special purpose or a general-purpose processor. Processor 1004 is connected to a communication infrastructure 1006 (for example, a bus, or network). Computer system 1000 may include a user input interface 1003 connected to one or more input device(s) 1005 and an output interface 1007 connected to one or more output devices 1009, which may be integrated input and display components. Input devices 1005 may include, for example, a connected device such as a mouse or touchpad, a keyboard, a touchscreen such as a resistive or capacitive touchscreen, etc.

Computer system 1000 also includes a main memory 1008, preferably random-access memory (RAM), and may also include a secondary memory 1010. Secondary memory 1010 may include, for example, a hard disk drive, a removable storage drive, flash memory, a memory stick, and/or any similar non-volatile storage mechanism. As will be appreciated by persons skilled in the relevant art(s), removable storage may include a non-transitory computer usable storage medium having stored therein computer software and/or data.

In alternative implementations, secondary memory 1010 may include other similar means for allowing computer programs or other instructions to be loaded into computer system 1000. Such means may include, for example, a removable storage unit and an interface which allow software and data to be transferred from the removable storage unit to computer system 1000.

Computer system 1000 may also include a communications interface 1024 implemented for example at the operating system level to allow data to be transferred between computer system 1000 and external devices, for example as signals over a communication channel. Communications interface 1024 may include a modem, a network interface (such as an Ethernet card), a communications port, a PCMCIA slot and card, or the like.

Various aspects of the present invention, such as the program code modules described above, may be implemented by software and/or firmware (also called computer programs, instructions or computer control logic) to program programmable hardware, or hardware including special-purpose hardwired circuits such as application-specific integrated circuits (ASICs), programmable logic devices (PLDs), field-programmable gate arrays (FPGAs), etc. of the computer system 1000, or a combination thereof. Computer programs for use in implementing the techniques introduced here may be stored on a machine-readable storage medium and may be executed by one or more general-purpose or special-purpose programmable microprocessors. The terms "computer program medium", "non-transitory computer readable medium" and "computer usable medium" introduced herein can generally refer to media such as removable storage unit, removable storage unit, and a hard disk installed in hard disk drive. Computer program medium, computer readable storage medium, and computer usable medium can also refer to memories, such as main memory 1008 and secondary memory 1010, which can be memory semiconductors (e.g. DRAMs, etc.). These computer program products are means for providing software to computer system 1000.

Computer programs are stored in main memory 1008 and/or secondary memory 1010. Computer programs may also be received via communications interface 1024. Such computer programs, when executed, enable computer system 1000 to implement the present invention as described herein. In particular, the computer programs, when executed, enable processor 1004 to implement the processes of embodiments of the present invention as described above. Accordingly, such computer programs represent controllers of the computer system 1000. Where the invention is implemented using software, the software may be stored in a computer program product and loaded into computer system 1000 using removable storage or communications interface 1024.

### Alternative Embodiments

The descriptions of the various embodiments have been presented for purposes of illustration, but are not intended to be exhaustive or limited to the embodiments disclosed. Many modifications and variations will be apparent to those of ordinary skill in the art without departing from the scope and spirit of the described embodiments.

Aspects of the present embodiments may be embodied as a system, apparatus, method or computer program product. Accordingly, aspects of the present disclosure may take the form of an entirely hardware embodiment, an entirely software embodiment (including firmware, resident software, micro-code, etc.) or an embodiment combining software and hardware aspects that may all generally be referred to herein as an "engine", a "module," a "system," or a "computer." In addition, any hardware and/or software technique, process, function, component, engine, module, or system described in the present disclosure may be implemented as a circuit or set of circuits. Furthermore, aspects of the present disclosure may take the form of a computer program product embodied in one or more computer readable medium(s) having computer readable program code embodied thereon.

Aspects of the present disclosure are described above with reference to flowchart and/or block diagrams of methods, apparatus (systems) and computer program products according to embodiments of the disclosure. It will be understood that each block of the flowchart and/or block diagrams, and combinations of blocks in the flowchart and/or block diagrams, can be implemented by computer program instructions. These computer program instructions may be provided to a processor of a general-purpose computer, special purpose computer, or other programmable data processing apparatus to produce a machine. The instructions, when executed via the processor of the computer or other programmable data processing apparatus, enable the implementation of the functions/acts specified in the flowchart and/or block diagram block or blocks. Such processors may be, without limitation, general purpose processors, special-purpose processors, application-specific processors, or field-programmable gate arrays.

The flowchart and block diagrams in the figures illustrate the architecture, functionality, and operation of possible implementations of systems, methods and computer program products according to various embodiments of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the blocks of the flowchart diagrams may occur out of the order noted in the figures. For example, two blocks shown in succession may, in fact, be executed substantially concurrently, or the blocks may sometimes be executed in the reverse order, depending upon the functionality involved. It will also be noted that each block of the flowchart diagrams, and combinations of blocks in the flowchart diagrams, can be implemented by special purpose hardware-based systems that perform the specified functions or acts, or combinations of special purpose hardware and computer instructions.

While the preceding is directed to embodiments of the present disclosure, other and further embodiments of the disclosure may be devised without departing from the basic scope thereof, and the scope thereof is determined by the claims that follow.

## Claims

1. Apparatus for recording a rehabilitation exercise in mixed reality, MR, the apparatus comprising:
a computer system (3) having a memory (33) and one or more processors (31);
a tracking camera (2), connected to the computer system (3), for tracking a position of at least a part of a user performing the rehabilitation exercise in a predefined space; and
a user input device (8, 50) for providing user input to the computer system (3);
wherein the computer system (3) is arranged to:
record one or more virtual objects (12) at one or more corresponding locations within the predefined space, said locations corresponding to said positions of at least part of the user when a user input is received from the user input device (8, 50); and
store exercise data (34) representing at least the positions of the one or more virtual objects (12) in the predefined space, as an exercise record representing a rehabilitation exercise to be performed by a patient.

2. Apparatus of claim 1, including a display (1) connected to the computer system (3) and arranged to display in mixed reality a video feed of the user within the predefined space together with the one or more virtual objects (12).

3. Apparatus of any preceding claim, including an MR headset (5) connected to the computer system (3) and arranged to display in mixed reality a view of the predefined space together with the one or more virtual objects (12).

4. Apparatus of claim 3, wherein the user input device comprises a headset camera (50) arranged to detect a user gesture.

5. Apparatus of any one of claims 1-3, wherein the user input device comprises a wearable or handheld controller (8) including indicia which are trackable by the tracking camera (2) so as to track the position of a part of the user.

6. Apparatus of any preceding claim, wherein said one or more tracking cameras (2) include a depth-sensing camera for tracking a 3D position of at least a part of a user performing the rehabilitation exercise in the predefined space.

7. Apparatus of any preceding claim, including a user interface (13) arranged to configure appearance and/or interactive properties of said one or more virtual objects (12); wherein said interactive properties may include one or more of:
a movement property indicating how the virtual object (12) moves in response to interaction therewith by a patient;
an existence property indicating one or more conditions under which the virtual object (12) is apparent to a patient; and
an interacting body part property indicating which body part or body parts of the patient are to interact with the virtual object (12).

8. Apparatus of any preceding claim, wherein the computer system (3) is arranged to record video content of the user performing the rehabilitation exercise in a predefined space, the video content being associated with the stored exercise data (34).

9. Apparatus for reproducing an interactive rehabilitation exercise record in mixed reality, MR, wherein the exercise record comprises exercise data (34) representing one or more positions of one or more virtual objects (12) in a predefined space for specifying a performance of the rehabilitation exercise by a patient;
the apparatus comprising:
a computer system (3) having a memory (33) and one or more processors (31); and
a tracking camera (2), connected to the computer system (3),
wherein the computer system (3) is arranged to:
display (S2-1) to the patient in mixed reality at least one of the virtual objects (12) at the corresponding position thereof;
track (S2-2) a position of at least a part of a patient performing the rehabilitation exercise in the predefined space;
detect (S2-3) whether said part of the patient has reached the position of one or more of the virtual objects (12) in mixed reality and if so:
store or update (S2-4) patient monitoring data recording that the part of the patient has reached the position of one or more of the virtual objects (12).

10. Apparatus of claim 9, wherein the computer system (3) is further arranged to provide feedback to the patient indicating that the part of the patient has reached the position of one or more of the virtual objects (12).

11. Apparatus of any one of claims 9-10, including a monitoring device (9) connectable to the computer system (3) for remote monitoring of the rehabilitation exercise performed by a patient; wherein the monitoring device (9) may be connectable to a plurality of said computer systems (3) for monitoring rehabilitation exercises performed by a plurality of patients at different locations; and/or wherein the monitoring device (9) may be arranged to modify configuration data of the one or more virtual objects (12).

12. Apparatus of any one of claims 9-11, wherein either the exercise data (34) includes configuration data indicating which body part of the patient is required to reach one or more of the corresponding virtual objects (12), or the computer system (3) is arranged to identify which part of the patient is required to reach the position of one or more of the virtual objects.

13. A method of recording a rehabilitation exercise in mixed reality, MR, using the apparatus of any one of claims 1-8, the method comprising:
recording one or more virtual objects (12) at one or more corresponding locations within the predefined space, said locations corresponding to said positions of at least part of the user when a user input is received from the user input device (8, 50); and
storing exercise data (34) representing at least the positions of the one or more virtual objects (12) in the predefined space, as an exercise record representing a rehabilitation exercise to be performed by a patient.

14. A method of reproducing an interactive rehabilitation exercise record (34) in mixed reality, MR, using the apparatus of any one of claims 9-12; the method comprising:
displaying to the patient in mixed reality at least one of the virtual objects (12) at the corresponding position thereof;
tracking a position of at least a part of a patient performing the rehabilitation exercise in the predefined space;
detecting whether said part of the patient has reached the position of one or more of the virtual objects (12) in mixed reality and if so:
storing patient monitoring data recording that the part of the patient has reached the position of one or more of the virtual objects (12).

15. A computer-readable storage medium comprising instructions which, when executed by a computer, cause the computer to carry out the method of claim 13 or claim 14.
